# EUROPEAN PATENT APPLICATION

(11) **EP 3 128 056 A2**
(43) Date of publication of application: **08.02.2017**
(21) Application number: 14872523.7
(22) Date of filing: 18.12.2014
(51) Int. Cl.: D04H 1/00

(54) **SOFT NON-WOVEN CLOTH FOR USE AS AN OUTER LAYER OF DISPOSABLE ABSORBENT ITEMS**

(30) Priority: 19.12.2013 MX 2013015294
(71) Applicant: Grupo P.I. Mabe, S.A. de C.V., 72230 Puebla (MX)
(72) Inventor: JUAREZ MANCERA, José Luis, Puebla 72230 (MX); CANALES ESPINOSA DE LOS MONTEROS, Carlos, Puebla 72230 (MX); SÁNCHEZ FERNÁNDEZ, Lucía del Carmen, Puebla 72230 (MX)
(74) Representative: Tari Lazaro, Aida
(86) International application number: PCT/IB2014/067048
(87) International publication number: WO 2015/092722

(57) **Abstract**

The invention relates to a non-woven cloth to be laminated to a polyethylene layer, such that
a. it is formed by a mixture of hydrophobic and hydrophilic polypropylene fibres;
b. it is produced by means of a carding process followed by thermal bonding;
c. during the thermal bonding, at least 15% of the area thereof is etched; and
d. it has a minimum base weight of 13 gr/m²

## Description

### FIELD OF THE INVENTION

The present invention refers to a non-woven fabric for use as an outer layer in disposable absorbent articles. Particularly, the present invention refers to a soft non-woven fabric, which has the appropriate mechanical properties to be laminated to a polyolefin layer, such as a polyethylene film, such that the resulting laminate is suitable to be used as an outer layer of a disposable absorbent article; the method of manufacturing thereof is also described below.

### BACKGROUND OF THE INVENTION

The disposable absorbent materials such as disposable diapers, disposable underwear, and feminine sanitary napkins, among others, have become an integral part of the everyday life of any family. Same are essentially comprised by an inner permeable layer, an outer impermeable layer, and an absorbent core provided there between; they may further comprise anti-leak barriers, elastic areas, adjustment systems and fastening systems. The main function of the outer layer of the disposal absorbent articles is to stop the fluids being absorbed by the absorbent core, protecting clothing that comes into contact with the article, so the impermeability is one of its main characteristics; in addition, it must be soft and pliable. Another desirable characteristic in an outer layer of a disposable absorbent article is that it has the appearance of cloth, both visual and tactile; to this end, it has become customary to use as an outer layer a laminate consisting of a polyolefin layer, such as low-density polyethylene, and a non-woven fabric layer, such that the polyolefin layer faces inward, contacting the absorbent core of the article, and the non-woven fabric layer faces outward. The first layer provides the laminate with the impermeability characteristic and contributes to the pliability and softness thereof, while the second layer provides it with the appearance and fabric-like tactile, further contributing with the softness and pliability of the outer layer of the disposable absorbent articles.

The non-woven fabric used for this type of laminate should have the appropriate mechanical properties for processing same in high-speed production lines, specifically, it must have certain longitudinal and cross-sectional strengths, elongations, specific weight, etc. Moreover, given that these laminate are to be used as an outer layer of a disposable absorbent article, the non- woven fabric, which will be the layer of the article visible to the consumer, should have a nice appearance and softness. Generally, this type of articles are packaged in polyethylene bags, such that when taking the product out of the bag, it is precisely taken out by the outer layer; so it is very important for this outer layer to transmit to the consumer or the retailer, at first contact, the idea of softness and comfort that the product will provide to the user. Moreover, as the disposable absorbent products are used for a short time only, they are disposed off and should be replaced with another equal or similar article, a very important aspect to consider in any element which forms a part thereof is the cost. Thus, the non-woven fabric of the present invention, in addition to having the appropriate mechanical properties, also has a nice appearance and softness; all this with a suitable cost so as to be used in consumable goods, such as disposable diapers.

So far, no non-woven fabrics exist yet in the market that meets all the requirements described in the foregoing paragraph. One may find non-woven fabrics with the appropriate strengths and base weights, but without the required softness or pliability, or in the other hand, they may have very good characteristics to the touch, but with high base weights, thereby increasing the cost of the product and rendering same non-suitable for the laminate processes, so may not be used in this kind of articles. Thus, there is still the need of developing a non-woven fabric that meets all the requirements described in the above paragraph in order to be used as the outer layer of a disposable absorbent article.

Some developments of non-woven fabrics that are used to be laminated onto the impermeable layer of a disposable absorbent article are described below:
U.S. patent US 20120179126 describes a non-woven fabric attached to a water-tight layer, the non-woven fabric comprises a first pattern of joined regions that define a second pattern of non-joined regions. The non-woven fabric described in the document cited has undergone a spun lacing process, and subsequently, a laminate-bonding process. The spunlacing is performed by water jets, that is, the non-woven fabric is passed through water jets followed by a dryer, this kind of treatment improves the softness, still, this process bears some disadvantages such as that it is complicated and the use of water in the process may involve a certain sanitary risk.

Patent applications US20120179125 and US20120177886 describe a non-woven fabric manufactured by "spun laid" fibers. These types of fibers are obtained from thermoplastic resins such as polypropylene, polyester, or nylon, these resins are thermally-bonded and are then extruded in fine threats forming long fibers that are subsequently used to produce the non-woven fabric in a humidifying process, these processes are expensive and complex. The non-woven fabric of the present invention is manufactured from short fiber and is manufactured by a dry-manufacturing process, significantly reducing the cost thereof.

U.S. patent application US20050095941 refers to a laminate which comprises directly extruding a plastic layer over a non-woven fabric to provide softness thereto, nevertheless, although different manufacturing processes and materials are cited, it is not clear in showing the non-woven fabric and the manufacturing process thereof.

The present invention describes a non-woven fabric with the appropriate mechanical characteristics to be attached, by any means known, to a layer of impermeable material such as polyethylene, forming a laminate, and with the required softness and appearance so that the laminate may be used as outer layer of a disposable absorbent article.

The laminate can be formed during the manufacturing process of the disposable absorbent article, which is known in the art as in-line laminate, or the laminate can be made in a separate process, so that the formed laminate enters into the manufacturing line of the disposable absorbent article. In any of the two cases, the layers forming the laminate can be attached by thermal bonding, ultrasonic bonding, or by means of adhesives or other suitable known means.

### OBJECTIVES AND ADVANTAGES OF THE INVENTION

The objective of the present invention is to provide a non-woven fabric with the appropriate mechanical properties in terms of base weight and horizontal and vertical strength, and with the appropriate appearance and softness, so as to be laminated with a polyolefin film layer, such that the laminate will be used as the outer layer in a disposable absorbent article.

A further objective of the invention is to provide a manufacturing method of the non-woven fabric.

Another objective of the invention is that the non-woven fabric has a cost that is suitable so as to be used in a disposable absorbent article.

Another further objective of the invention is that the non-woven fabric has an embossing pattern, which improves its softness, strength and provides a better appearance thereto.

### DETAILED DESCRIPTION OF THE INVENTION

The absorbent articles mentioned herein may be, but are not limited to: disposable diapers and underwear for babies, feminine sanitary napkins, adult diapers, underwear, and protectors, etc. These articles are used for a period of time that may vary depending on use, but generally from 2 to 4 hours during the day or 8 to 12 hours at night, then are disposed off and replaced by another article, such that a person uses 4 to 5 articles per day. Thus, an important aspect to consider in improving these articles is the cost involved in its manufacture, given that the buyers of these type of articles are not amenable to paying a high cost for a product that will be dicharged after some hours of use, and immediately shall be replaced for another one.

Focusing on the present invention, non-woven fabrics can be developed for use as an outer layer in disposable absorbent articles with optimum softness characteristics and appearance, but with high base weights which increase the cost and preclude its use in such products. In the present invention, a non-woven fabric is described that, due to its mechanical characteristics, softness and appearance, as well as its cost, is ideal for being attached to an impermeable film layer forming a laminate which will be used as an outer layer of disposable absorbent articles.

A disposable absorbent article is basically comprised by an inner permeable layer, an outer impermeable layer, and an absorbent core placed there between. Generally, a non-woven fabric is used as the inner layer, which allows the passage of fluids, the absorbent core is generally comprised by absorbent material fibers mixed with superabsorbent particulate material, and a film, which impedes the passage of fluids, is used as the outer layer. The disposable absorbent articles can additionally comprise other elements: fastener system, elastic areas, transfer layers, anti-leak barriers, elastic material at the waist and leg portions, etc.

The main function of the outer layer of the disposable absorbent articles is to stop the fluids absorbed by the absorbent core, protecting the clothing that comes into contact with the article, impermeability therefore is one its main characteristics; further, it should be soft and flexible. Another desirable characteristic in an outer layer of a disposable absorbent article is that it has the appearance of fabric, both visually and to the touch; for obtaining both characteristics, impermeability and fabric-like appearance, a laminate consisting of a plastic film such as a polyethylene and a non-woven fabric can be used, such that the polyethylene layer is placed facing inward, contacting the absorbent core, and the non-woven fabric facing outward. The first one provides the characteristic of impermeability and contributes to the pliability and softness, while the second one contributes to the appearance and fabric-like touch, further contributing to the softness and pliability of the outer layer of the disposable absorbent articles.

The non-woven fabric used for this type of laminate should have certain mechanical properties for withstanding the longitudinal and cross section strengths used during the lamination and during manufacturing process of the disposable absorbent articles, these mechanical properties are:
- Longitudinal strength: 1000 to 2500 gr/2.54 cm
- Cross-sectional strength: from 90 to 250 gr/2.54 cm
- Longitudinal elongation: 50 to 80 %
- Transverse elongation: 100 to 300 %

Moreover, it should have a base weight between 13 and 25 gr/m² that does not increase the cost of the final product and may be introduced in an industrial process of manufacture of the absorbent articles.

The non-woven fabric of the invention, in addition to having the cited mechanical characteristics and base weight, is softer than the fabrics with the same mechanical characteristics and base weight that are currently available in the market. This is achieved through a carding process followed by a thermal bonding process that follows the stages listed below:
1. Pre-opening of the fibers
2. Opening
3. Weighing, mixing and homogenization of the fibers in adequate ratios.
4. Formation of nappa
5. Weight control of nappa
6. Carding and combing of the nappa, formation of strands
7. Thermal bonding
8. Application of tension
9. Cutting and spooling

Each of these steps comprises a series of technical considerations that provide the non-woven fabric of the invention with its final characteristics. Each of these steps is carried out using the necessary means which are, but are not limited to, machinery, independent machinery or assembly systems for non-woven fabric.

A mixture of hydrophobic and hydrophilic polypropylene fibers is used in a ratio ranging from 90:10 to 10:90, these fibers shall have the following characteristics:

| Table 1 Fiber characteristics | | | |
|---|---|---|---|
| Parameter | Units | HYDROPHOBIC | HYDROPHILIC |
| Denier | gr/9000m | 2.2 ± 0.15 | 2.17 ± 0.15 |
| Tenacity | gr/De | 2.5 ± 0.15 | 1.94 ± 0.15 |
| Elongation | % | 370 ± 40 | 338 ± 19.02 |
| Curliness | Curls/cm | 6.0 ± 1 | 9.0 ± 1 |
| Length | mm | 40.0 ± 2.0 | 40.0 ± 2.0 |
| Cohesion | gr | 110.0 ± 10.0 | 110.0 ± 10.0 |

The hydrophobic fiber provides the fabric with liquid repellant, while the hydrophilic fiber helps to a better formation of the fibers at the time of the carding and combing of the fiber.

Once the fibers are weighed and homogenized, the "nappa" is formed, this "nappa" will result in the fabric; the "nappa" is formed with the right amount of fibers for obtaining the required base weight in the final product. Two or more films can be formed in order to obtain a homogenous non-woven fabric in respect to the distribution of the fibers, these films will then be thermally bonded.

To obtain the required softness, the most important step of the process is the thermal bonding process. This is done by passing the films through two rollers, a top roller with a special protrusion-shaped embossment, and a bottom smooth roller. The rollers operate at a temperature between 120 and 180°C; and a pressure of between 65 to 95 Kgf/cm² is applied there between, such that, when passing the films between the rollers, the fibers are thermally-bonded and the embossment of the top roller is engraved in the non-woven fabric. The preferred temperature for this step in the process is between 130 and 150°C and the preferred pressure between the rollers is between 70 and 80 Kgf/cm².

To achieve the required characteristics of softness and appearance, the pattern on the top roller should be such that the resulting non-woven fabric has:
- An engraving area of between 10% and 25%;
- An engraving density of between 8 to 12 images/cm²
- A maximum engraving depth of 0.65 mm

Finally, the fabric is tensioned by means of rollers comprising a tension spring or rollers with pneumatic tension controls, in order to provide the required tension for spooling and cutting..

In any case, the non-woven fabric resulting from the aforementioned process comprises a series of technical characteristics that make it ideal for the exterior of disposable absorbent articles, this is, softness to the touch, easy bonding to the impermeable layer and appropriate mechanical characteristics for a good processability during the laminating process. The following table outlines the mechanical properties of the non-woven fabric of the invention:

| Table 2 Non-woven fabric characteristics | |
|---|---|
| PROPERTY | MEASUREMENT |
| Basis weight | 13-15gr/m² |
| Longitudinal strength | 1000 - 2500 gr/2.54 cm |
| Cross-sectional strength | 90 - 250 gr/2.54 cm |
| Gauge | 0.10-0.2 mm |
| Longitudinal elongation | 50 - 80% |
| Cross-sectional elongation | 100 - 300% |

The softness of the non-woven fabric of the present invention was evaluated using a sensory test as follows:
- Surveyed persons were blindfolded
- Two disposable diaper samples were placed in front of them:
   o Sample A: disposable diaper with an outer layer comprised by a laminate consisting of the non-woven fabric of the invention and a low-density polyethylene; the non-woven fabric with a base weight of 17 g/m² ;
   o Sample B: disposable diaper with an outer layer comprised by a laminate consisting of the non-woven fabric of the invention now used in our products, with a base weight of 17 g/m² combined and the same low-density polyethylene as in Sample A.
      - The persons were asked to feel the outer layer of both Samples A and B with their hands and to indicate which one felt the softest.

The results were as follows:
o TOTAL NUMBER OF SURVEYED PERSONS: 50
o PERSONS WHO SAID SAMPLE "A" WAS THE SOFTEST TO THE TOUCH: 32 (64%)
o PERSONS WHO SAID THAT SAMPLE "B" WAS THE SOFTEST TO THE TOUCH: 18 (32%)

In accordance with the above, it can be concluded that the non-woven fabric of the invention is softer to the touch with a statistical feasibility of 90%.

While the invention has been described in terms of the preferred embodiments, it should be evident to those skilled in the art that variations and modifications thereof can be implemented without deviating from the scope of the present invention.

## Claims

1. A non-woven fabric for be bonded to a polyethylene layer to form a laminate, **characterized in that**:
a. it is comprised by a mixture of polypropylene hydrophobic and hydrophilic fibers;
b. it is manufactured by means of a carding process followed by thermal bonding
c. during the thermal bonding, the fabric is passed through two rollers applying heat and pressure thereto and is engraved in at least 15% of its area
d. has a minimum base weight of 13 gr/m².

2. A non-woven fabric as described in claim 1, **characterized in that** the mixture of hydrophilic and hydrophobic fibers ranges between 10:90 and 90:10.

3. A non-woven fabric as described in claim 1, **characterized in that**, during the thermal bonding process, a pressure between 18 and 25 tons is applied.

4. A non-woven fabric as described in claim 1, **characterized in that** the engraving density ranges between 8 and 12 images /cm².

5. A non-woven fabric as described in claim 1, **characterized in that** the thermal bonding is made between two rollers, a top roller and a bottom roller, such that the top roller has protrusions with the engraving pattern.

6. A non-woven fabric as described in claim 5, **characterized in that** the protrusions have a maximum height of 0.65 mm.

7. A non-woven fabric as described in claim 1, **characterized in that** it has a minimum longitudinal tension strength of 1000gr/2.54 cm.

8. A non-woven fabric as described in claim 1, **characterized in that** it has a minimum cross-sectional strength of 90 gr/2.54 cm.

9. A non-woven fabric as described in claim 1, **characterized in that** it has a minimum longitudinal elongation of 50%.

10. A non-woven fabric as described in claim 1, **characterized in that** it has a minimum cross-sectional elongation of 100%.
